# FASCICULE DE BREVET EUROPEEN

(11) **EP 2 395 942 B1**
(45) Date de publication et mention de la délivrance du brevet: **24.07.2013**
(21) Numéro de dépôt: 10708310.7
(22) Date de dépôt: 09.02.2010
(51) Int. Cl.: A61F 2/04, A61B 17/11, A61F 2/82

(54) **DISPOSITIF CHIRURGICAL APTE A REALISER LA PROTECTION TEMPORAIRE D'UNE ANASTOMOSE**
CHIRURGISCHE VORRICHTUNG ZUM ZEITWEISEN SCHUTZ VOR ANASTOMOSE
SURGICAL DEVICE FOR THE TEMPORARY PROTECTION OF ANASTOMOSIS

(30) Priorité: 10.02.2009 FR 0950819
(43) Date de publication de la demande: 21.12.2011
(73) Titulaire: Khosrovaninejad, Charam, 84210 Pernes Les Fontaines (FR)
(72) Inventeur: Khosrovaninejad, Charam, 84210 Pernes Les Fontaines (FR)
(74) Mandataire: Domange, Maxime
(86) Numéro de dépôt international: PCT/FR2010/050210
(87) Numéro de publication internationale: WO 2010/092291

(56) Documents cités:
- WO-A-2007/045229
- US-A1- 2007 032 879
- US-A1- 2008 208 357

## Description

La présente invention concerne un dispositif chirurgical apte à réaliser la protection temporaire d'une anastomose sur le côlon, le rectum ou le canal anal.

L'objet de l'invention est donc un dispositif permettant de protéger l'anastomose (union) réalisée entre les deux extrémités d'un segment d'intestin, pour prévenir ou amoindrir les risques de désunion appelée fistule anastomotique. Les fuites occasionnées peuvent générer des infections, des abcès, ou des péritonites, cause de complications et de mortalité postopératoires importantes.

Le moyen utilisé à ce jour consiste à procéder à une dérivation du flux intestinal en amont de la zone d'anastomose. On procède par l'extériorisation, à travers la paroi abdominale, d'un segment de l'intestin situé en amont de l'anastomose. Cela s'appelle une stomie digestive. Le bol fécal est alors recueilli à l'extérieur de l'abdomen, dans un sac collé sur la paroi abdominale, autour de l'intestin, encore appelé « poche »: Cette technique permet de dériver le bol fécal en amont de l'anastomose au prix d'une stomie. Mais la stomie a des répercussions socioprofessionnelles considérables ainsi qu'un taux significatif de complications locales (cutanées et intestinales) ou générales (déshydratation). Par ailleurs, la fermeture de la stomie, 6 à 8 semaines plus tard, nécessite une nouvelle intervention chirurgicale comportant une morbidité non négligeable. Enfin, le dispositif appelé « poche » et les soins de stomie ont un coût important.

On connaît une alternative à la stomie, dénommée « bypass ». Dans ce procédé, il n'y a pas de dérivation digestive. La zone anastomotique est protégée du contact fécal au moyen d'une interface. L'interface est un tube très souple (en vinyle par exemple), introduit dans la lumière de l'intestin et solidarisé à l'extrémité proximale de celui-ci par du fil résorbable, en amont de la future zone d'anastomose. L'autre extrémité du tube est laissée libre dans la lumière de l'intestin distal. Puis, on réalise l'anastomose. Après un délai moyen de 10 jours nécessaire pour la bonne cicatrisation de l'anastomose, et une fois les fils de fixation résorbés, le tube s'élimine spontanément par le bol fécal, à travers le canal anal.

Ce procédé a été décrit depuis de nombreuses années et un dispositif a été même commercialisé depuis 1992, sous le nom de COLOSHIELD^{®}. Les travaux expérimentaux et cliniques ont confirmé la fiabilité de ce dispositif. Mais, ce produit n'est plus utilisé par les équipes chirurgicales depuis de nombreuses années, car il présente un certain nombre d'inconvénients.

L'un des inconvénients du dispositif Coloshield^{®} consiste dans sa solidarisation avec la lumière de l'intestin, en amont de la future zone d'anastomose. Cela oblige à des manoeuvres d'éversion de l'extrémité proximale de l'intestin afin de présenter sa face interne et y fixer le tube par des points de fil résorbable. Cette manoeuvre est source de tractions, de plaies ou de déchirure de l'intestin à l'endroit même où ce dernier devra s'unir avec l'extrémité distale de l'intestin. Un autre inconvénient avec ce dispositif est l'impossibilité d'utiliser une agrafeuse circulaire automatique pour la confection de l'anastomose. Or, plus de 80% des anastomoses colorectales sont confectionnées grâce à ces machines.

On connaît dans WO 03/094785, un élément d'ancrage permanent de forme conique ou en entonnoir réalisé en matière plastique muni d'une valve implanté de manière permanente au début du tube digestif notamment au niveau de l'intestin grêle, et couplé à une gaine souple s'étendant dans l'intestin grêle en aval dudit élément d'ancrage, ce dispositif étant destiné au traitement de l'obésité. Ledit élément d'ancrage présente un diamètre externe légèrement supérieur à celui de l'intestin même en phase dilatée lors du transit intestinal, car ledit élément d'ancrage doit rester fixé à l'estomac de manière permanente par des sutures.

On connaît également dans US 2008/0208357 une méthode de traitement curatif de fistule, c'est-à-dire de fuite du contenu d'intestin au niveau d'anastomose gastro-intestinale, comprenant la mise en oeuvre d'un élément d'ancrage consistant en un « stent » de 25 à 45mm de longueur et 20 à 40mm de diamètre couplé à une gaine souple de courte longueur permettant de réparer et colmater la fistule ou fuite. Ce « stent » est introduit dans l'oesophage par la voie buccale et implanté au-dessus de la zone de rétrécissement anatomique de jonction entre l'oesophage et l'estomac. Le «stent » ainsi implanté dans l'oesophage ne doit pas migrer et, en tout état de cause, est empêché de migrer de par son implantation en amont de la zone de rétrécissement anatomique de jonction entre l'oesophage et l'estomac.

Dans US 2008/0208357 la longueur de la gaine est nécessairement inférieure à la longueur de l'anse jéjunale anastomosée à l'estomac (environ 60 cm). Le dispositif doit nécessairement être introduit à partir de la cavité buccale comme mentionné. Une fois le stent mis en place, la gaine doit être poussée en aval du stent pour être déployée dans l'anse jéjunale. Au total la distance qui sépare la cavité buccale de l'extrémité distale de la gaine déployée est de 57 à 72 cm. En pratique, il ne serait pas possible de déployer en la poussant une gaine de longueur supérieure à de 15 à 30cm. De plus, ces manoeuvres de poussée de la gaine sont dangereuses pour l'anastomose car elles s'effectuent sur une zone très fragilisée par la fistule, et elles peuvent aggraver l'état de la fistule.

Enfin dans US 2008/0208357 l'épaisseur de la gaine est très fine, de 0.01 à 0.025 mm, car dans cette application la gaine ne doit pas irriter l'oesophage, qui est particulièrement sensible. Mais, en raison de sa finesse, une telle gaine ne peut avoir la mémoire de forme. Elle peut subir une torsion ou des plicatures. C'est pourquoi ce brevet fait référence à un autre brevet US-7267794 pour la mise en oeuvre d'un dispositif en forme de fil métallique circulaire qui empêche la torsion de la gaine.

Le problème formulé selon la présente invention est de fournir un dispositif de protection temporaire d'anastomose du type « bypass » au niveau du côlon, dont la mise en place intervient après la confection de l'anastomose et ce quelle qu'en soit la technique (fil de suture ou agrafeuse), qui ne nécessite pas de manoeuvres de fixation et qui s'élimine spontanément sans intervention du chirurgien, après un délai suffisant après la cicatrisation de l'anastomose.

Pour ce faire, la présente invention fournit un dispositif chirurgical apte à réaliser la protection temporaire d'une anastomose sur le côlon, le rectum ou le canal anal comprenant :
a) un élément longitudinal creux semi-rigide dit élément d'ancrage temporaire dont la paroi dite première paroi, définit une surface de révolution autour d'un axe longitudinal XX comprenant une partie courante sensiblement cylindrique à section sensiblement circulaire, ladite première paroi présentant un diamètre externe que l'on peut faire varier de façon contrôlée entre :
   - un diamètre externe réduit D1' en position radiale rétractée de ladite première paroi d'au plus 10mm, et
   - un diamètre externe maximal D1 en position d'expansion radiale maximale de la partie courante de ladite première paroi, de 20 à 40 mm, de préférence de 25 à 33 mm, et
b) une gaine à paroi tubulaire souple fixée au dit élément d'ancrage contre, de préférence autour de ladite première paroi,
caractérisé en ce que :
1) ladite première paroi dudit élément d'ancrage temporaire comprend une dite partie courante de plus grand diamètre, de longueur L1 d'au moins 50 mm, de préférence de 70 à 150 mm, et
2) ladite gaine présente au repos une longueur L3 en aval dudit élément d'ancrage d'au moins 50 cm, de préférence au moins lm, et un diamètre externe de 20 à 40 mm, de préférence de 25 à 33mm, et ladite gaine est en matériau élastomère biocompatible d'épaisseur de paroi de 0,05 à 1mm.

Le diamètre externe réduit D'₁ en position radiale rétractée de ladite première paroi permet d'introduire ledit élément d'ancrage dans cette position radiale rétractée, par voie anale puis l'acheminer dans le côlon jusqu'à une position d'ancrage temporaire en amont d'une anastomose sur le côlon, le rectum ou le canal anal. Et, le diamètre externe maximal D₁ en position d'expansion radiale maximale de ladite paroi permet que ledit élément d'ancrage reste ancré temporairement, fixé par la force d'expansion radiale, contre la paroi de l'intestin, en l'absence de transit intestinal, et soit apte à être libéré et commencer à migrer lorsque l'intestin se contracte et se dilate lors de ce qu'on appelle « phase de péristaltisme », à la reprise du transit intestinal.

S'agissant dudit élément d'ancrage, on entend par :
- « semi-rigide », que ladite première paroi dudit élément d'ancrage se maintient dans chacune de ces positions rétractées ou en expansion dans une forme donnée définissant une dite surface de révolution, et
- « diamètre externe que l'on peut faire varier de façon contrôlée », que le diamètre de ladite première paroi peut varier de façon déterminée en fonction de conditions de mise en oeuvre du dispositif, que ce soit des paramètres tels que la température, et/ou des moyens mécaniques indépendants coopérant avec ledit élément d'ancrage tel qu'un instrument de pose, notamment un « introducteur », comme explicité ci-après.
On comprend que ledit élément d'ancrage peut adopter:
- un diamètre externe réduit D'₁, de ladite première paroi au moins inférieur à celui de l'intestin au repos, de préférence inférieur à 10mm, de manière à être apte à être introduit par voie anale jusqu'en amont d'une anastomose dans l'intestin, et
- un diamètre externe en expansion radiale maximale de ladite première paroi D₁ supérieur ou égal à celui de l'intestin au repos de manière à pouvoir être ancré temporairement par appui sur la paroi intestinale par expansion radiale au moins sur une partie dudit élément d'ancrage, et D₁ étant toutefois inférieur au diamètre maximale de l'intestin en travail en phase péristaltique de telle sorte que ledit élément d'ancrage reste fixé contre la paroi de l'intestin en l'absence de transit intestinal, notamment pendant la phase de paralysie intestinale post opératoire dite iléus, mais ne soit plus fixé à l'intestin et en pratique commence à migrer lors de la reprise du transit intestinal par glissement le long de la paroi intestinale.

Un moyen classique pour déterminer D₁ est le calibrage de la section d'une des extrémités de l'intestin ou moyen d'instruments appelés « bougies », couramment utilisés par les chirurgiens. Un autre moyen pour déterminer D₁ est de le considérer comme égal au diamètre de l'agrafeuse utilisée pour la confection de l'anastomose mécanique le cas échéant. Dans ce cas D₁ peut plus particulièrement varier de 25 à 33 mm, correspondant au diamètre externe des agrafeuses actuellement disponibles dans le commerce.

On entend par « extrémité longitudinale », une extrémité dans la direction longitudinale dudit élément d'ancrage ou le cas échéant de ladite gaine.

On entend par longueur et diamètre de la gaine « au repos », ses longueur et diamètre lorsque son élasticité longitudinale et respectivement radiale ne sont pas sollicitées.

Après la reprise du transit intestinal, le péristaltisme de l'intestin provoque la libération et migration dudit élément d'ancrage ainsi libéré, mais les propriétés d'expansion radiale de l'élément d'ancrage permettent à sa paroi externe de continuer à glisser au contact de la paroi interne de l'intestin et d'obtenir ainsi une étanchéité suffisante pour empêcher le passage du bol fécal entre la paroi externe de l'élément d'ancrage et la paroi interne de l'intestin et ainsi de continuer à protéger l'anastomose. En effet, le bol fécal doit passer impérativement à l'intérieur de la gaine et reste bien séparé des parois de l'intestin au niveau de l'anastomose.

Les valeurs ci-dessus de diamètre en expansion radiale de l'élément d'ancrage correspondent à des valeurs légèrement supérieures au diamètre de l'intestin au repos selon les individus, mais inférieure à la valeur 30 à 60 mm de diamètre maximal de l'intestin dilaté lors du transit intestinal. En outre la longueur de ladite partie courante est telle que la surface de contact entre ledit élément d'ancrage et la paroi du côlon combiné à la force d'expansion radiale, sont telles que ledit élément d'ancrage en position d'expansion maximale soit apte à rester fixé temporairement par sa force expansion radiale contre la paroi de l'intestin en amont de l'anastomose et ne migre pas pendant au moins 3 jours de préférence 5 jours après son ancrage en l'absence de transit intestinal. A défaut d'une longueur d'au moins 50mm de partie courante en contact contre le côlon, ledit élément d'ancrage ne pourrait pas rester ancré au moins 3 jours sans avoir recours à des moyens de fixation, nécessitant alors des moyens et/ou interventions pour désactiver ses moyens de fixation et permettre sa migration , ou sans avoir recours à des élément d'ancrage présentant un diamètre d'expansion maximale plus important ce qui pourrait blesser le côlon et de surcroît empêcherait la libération naturelle de l'élément d'ancrage lors de la reprise du transit.

D'autre part, la longueur de la gaine est telle que la distance entre l'anastomose et le site d'ancrage en amont peut être d'au moins 50 cm, de préférence au moins 1 m, et ledit élément d'ancrage peut être ancré dans le côlon suffisamment en amont de l'anastomose pour que lors de la reprise du transit , son temps de migration entre ladite position d'ancrage et ladite anastomose soit d'au moins 3 jours de préférence au moins 6 jours, et que ladite gaine protège l'anastomose, voire dépasse de l'orifice anal lorsque ledit élément d'ancrage est en position d'ancrage.

Ainsi, dans la mesure où la phase de paralysie intestinale post opératoire dite iléus dure de 3 à 5 jours, il est ainsi possible de protéger l'anastomose de cette même période additionnée du temps que met ledit élément d'ancrage pour migrer après libération entre son site d'ancrage en amont de l'anastomose et le site de l'anastomose, étant entendu que ce temps de migration dépend de la distance à parcourir entre le site d'ancrage et l'anastomose. En pratique une distance de 50cm à 1m se traduit par un temps de migration de 3 à 6 jours, de sorte qu'au total l'anastomose sera protégée pendant une durée d'au moins 6 à 11 jours après la réalisation de l'anastomose.

On comprend que ladite gaine de par sa constitution en élastomère présente des propriétés d'étirabilité radiale et longitudinale similaires à celle de la paroi intestinale, propriétés qui sont celles d'un matériau élastomère en forme de dite gaine, celui-ci présentant des propriétés d'élasticité radiale et longitudinale. Ces propriété d'élasticité radiale et longitudinale de la gaine sont similaires à celles de la paroi du côlon et permettent que le transit intestinal se fasse correctement dans ladite gaine pendant toute la durée de migration de l'élément d'ancrage soit une durée d'au moins 6 à 10 jours.

L'élasticité longitudinale de la gaine élastomère peut être plus importante que celle de l'intestin, sans que cela ne pose de difficulté, au contraire elle présente l'avantage de pouvoir être tirée sur la partie de gaine en dépassement anal, de manière à la couper et à ce qu'elle se rétracte à l'intérieur en amont dans le rectum.

De par son élasticité radiale, ladite extrémité longitudinale de la gaine reste fixée à ladite extrémité dudit élément d'ancrage quelque soit son niveau d'expansion radiale.

D'autre part, les caractéristiques d'épaisseur de la gaine combinée à son élasticité lui confèrent une propriété de mémoire de forme. On entend ici par « propriétés de mémoire de forme » que le matériau élastomère constitutif de ladite gaine retrouve naturellement sa forme initiale lorsqu'il est déformé par pliage. Compte-tenu de la grande longueur de la gaine, ces propriétés de mémoire de forme sont importantes, pour que le matériau retrouve naturellement sa forme longitudinale sans créer de blocage du transit en cas de plicatures de la gaine lesquelles peuvent effectivement intervenir pendant sa migration après libération de l'élément d'ancrage.

Dans un mode préféré de réalisation, ladite partie courante de dite première paroi s'étend depuis l'extrémité longitudinale amont de ladite première paroi jusqu'à une partie terminale avale profilée de diamètre plus petit que celui de la partie courante cylindrique, le diamètre externe en expansion radiale maximale D2 de l'extrémité avale de ladite première paroi étant de 20 à 35 mm, et la longueur L2 de ladite partie terminale profilée de la première paroi étant de 10 à 30 mm, de préférence de 15 à 25 mm, de préférence le diamètre de ladite partie terminale diminuant progressivement entre ladite partie courante et ladite extrémité avale de ladite première paroi.

La partie avale profilée de l'élément d'ancrage permet de faciliter sa migration à travers la zone d'anastomose laquelle présente en général une réduction de diamètre de passage par rapport au côlon en amont et en aval de la zone d'anastomose, de façon à éviter un blocage à ce niveau. Cette réduction de diamètre est due à une sténose au niveau de l'anastomose. En revanche, une partie amont profilée de l'élément d'ancrage entrainerait un risque de fuite entre l'élément d'ancrage et la paroi de l'intestin.

Plus particulièrement, la réduction de diamètre de ladite partie terminale avale profilée est obtenue en entourant celle-ci d'un anneau en matériau élastomère de diamètre inférieur au dit diamètre d'expansion maximale radiale de ladite partie courante.

De préférence encore :
- ledit élément d'ancrage est réalisé en un matériau lui conférant des propriétés d'élasticité radiale de sorte qu'il peut être comprimé radialement en dite position rétractée et adopter une dite position d'expansion radiale maximale après relâchement de la compression radiale, et
- ladite gaine est réalisée en matériau à base d'élastomère polymérique biocompatible du type silicone ou polyuréthane d'épaisseur de paroi de 0,1 à 0,5 mm et présentant des propriétés d'élasticité radiale et longitudinale, des propriétés de mémoire de forme et propriétés de non collabilité.

On entend par « propriétés de non collabilité » que le matériau élastomère constitutif de ladite gaine présente un coefficient d'adhérence tel que les deux surfaces opposées de paroi interne de la gaine ne se collent pas l'une contre l'autre lors de pliement, ceci pour ne créer aucune résistance au passage des gaz et matières.

Ledit élément d'ancrage peut être maintenu en position radiale rétractée avec un instrument dénommé « introducteur » décrit ci- après, l'expansion radiale intervenant après dégagement de l'élément d'ancrage par rapport à l'introducteur.

On comprend aussi que :
- ladite gaine présente un diamètre au repos sensiblement au moins égal au dit diamètre externe réduit D'₁ en rétractation radiale dudit élément d'ancrage creux, et inférieur à celui de l'intestin au repos, de préférence ledit diamètre au repos de ladite gaine est sensiblement égal à celui de la paroi intestinale au repos, et
- ladite gaine s'étend en aval de l'extrémité dudit élément d'ancrage à laquelle elle est fixée sur une longueur correspondant à la distance entre la position d'ancrage en amont de ladite anastomose et une position en aval de préférence jusqu'à l'orifice anal.

Plus particulièrement, l'épaisseur de ladite première paroi de l'élément d'ancrage est de 0,5 à 5 mm, de préférence de 1 à 3 mm.

L'élasticité longitudinale de la gaine élastomère peut être plus importante que celle de l'intestin, ce qui ne pose pas de difficulté, mais au contraire présente l'avantage de pouvoir tirer sur le dépassement anal de la gaine le cas échéant, de manière à pouvoir la couper et qu'ainsi elle se rétracte à l'intérieur du rectum ou du canal anal.

Ladite première paroi dudit élément d'ancrage peut être pleine ou ajourée, notamment par des pores ou mini perforations.

Dans un mode de réalisation particulier, ledit élément longitudinal creux d'ancrage temporaire est une prothèse entérale du type stent dont la paroi tubulaire est recouverte sur au moins sa face externe d'un revêtement en matériau synthétique biocompatible, de préférence un élastomère de type silicone ou polyuréthane.

Ce revêtement externe est doublement avantageux en ce qu'il permet de faciliter le largage dudit élément d'ancrage par glissement le long de la paroi intestinale à la reprise du transit et pendant l'ancrage protège la paroi intestinale contre laquelle ledit élément longitudinal est en expansion ce qui pourrait risquer de créer une incrustation dans le tissu de la paroi et empêcher le re-largage subséquent, voir créer une perforation de la paroi intestinale.

Ces prothèses entérales du type « stent » sont utilisées dans les tumeurs intestinales depuis une vingtaine d'années, principalement dans le traitement palliatif des sténoses (rétrécissements) tumorales de l'oesophage, du duodénum et du côlon. Elles n'ont jamais été conçues comme moyen d'ancrage temporaire d'un dispositif servant à protéger une anastomose intestinale selon la présente invention. Autrement dit, une prothèse entérale solidarisée à un manchon souple, le tout conçu dans ces caractéristiques dimensionnelles et notamment d'élasticité telle que définies ci-dessus pour protéger une anastomose digestive, n'a jamais été proposée.

De préférence, ledit élément d'ancrage temporaire est une prothèse entérale dont ladite première paroi est formée par un maillage de fils spiralés métalliques ou en matériau élastomère, notamment à base de silicone, de préférence revêtus par une couche d'un matériau synthétique biocompatible qui recouvre lesdites mailles, ledit revêtement étant de préférence en matériau élastomère biocompatible, tel que du silicone.

De façon connue, l'expansion radiale résulte alors du croisement des fils métalliques dont la variation angulaire permet de varier la largeur du losange ou parallélogramme des mailles dudit maillage de fils spiralés.

Avantageusement, ledit élément d'ancrage est réalisé en un matériau qui lui confère une dite expansion par élasticité radiale uniquement à température au moins égale à la température ambiante de 20°C, notamment à la température du corps humain, ledit élément d'ancrage étant en dite position radiale rétractée à une température inférieure à ladite température ambiante, de préférence inférieure à 5°C. On comprend que le matériau en forme tubulaire change de diamètre automatiquement en fonction de la température ambiante.

Plus particulièrement encore, ledit élément d'ancrage est une prothèse entérale dont ladite première paroi est formée par un maillage de fils spiralés, de préférence en nitinol.

Le nitinol est un alliage métallique présentant des propriétés d'expansion radiale progressive en fonction de la température, à température supérieure ou égale à la température ambiante (25°C), ce qui permet de le conserver sous forme rétractée à température plus froide, notamment à 4°C au stockage. Une fois rétracté à basse température, il reste ainsi rétracté suffisamment de temps pour pouvoir le loger dans le tube introducteur et l'acheminer dans l'intestin à l'aide dudit introducteur. Une fois libérée dans l'intestin, la prothèse retrouve progressivement son expansion radiale sous l'effet d'une température ambiante plus élevée, celle du corps humain.

Dans un mode de réalisation particulier, la liaison entre ladite gaine et l'élément d'ancrage se fait de manière à ce que l'extrémité de ladite gaine adhère et recouvre élastiquement au moins la surface de l'extrémité longitudinale du dit élément longitudinal creux.

Dans une variante de réalisation préférée, ladite gaine adhère à et recouvre élastiquement la surface externe dudit dit élément d'ancrage, au moins sur la surface externe de l'extrémité avale dudit élément d'ancrage. Ladite gaine fait ainsi fonction de revêtement recouvrant les mailles de l'élément d'ancrage le cas échéant.

Les deux modes de réalisation qui précèdent peuvent être réalisés en mettant en oeuvre pour la fabrication de la gaine, un procédé de trempage d'un tube plein, le cas échéant en prolongation d'un dit élément d'ancrage, dans un bain de dite composition polymérique sur lequel ledit matériau polymérique vient se mouler, ledit élément longitudinal creux étant enfilé autour dit tube plein de moulage avant trempage.

Pour satisfaire les propriétés d'expansion radiale de la gaine élastomère, compte tenu des épaisseurs mises en jeu (0,05 à 0,4 mm), il est nécessaire de mettre en oeuvre des compositions élastomères présentant une certaine dureté, ainsi que collabilité (coefficient d'adhérence), mémoire de forme (DRC- degré de déformation rémanente à la compression), dont les fourchettes de valeur sélectionnée sont explicitées ci-après.

Plus particulièrement, ladite gaine est réalisée en matériau polymérique biocompatible à base d'élastomère de préférence du type silicone et/ou polyuréthane présentant les propriétés suivantes :
- une dureté de 5 à 95 Shore A, de préférence de 50 à70 Shore A,
- une déformation rémanente à la compression, ou DRC de 10% à 35%, de préférence de 17% à 23%,
- un coefficient de friction de 0,30 à 0,90, de préférence de 0,35 à 0,45.

On entend par « dureté » l'énergie de déformation élastique d'un élastomère exprimé en Shore A, selon la norme DIN 53505 par exemple.

On entend par « DRC », le degré de déformation résiduelle après libération d'une charge sur un échantillon élastomère après un temps déterminé selon le test de norme DIN 53517 ISO 815B par exemple la propriété de mémoire de forme à la pliure , le matériau reprenant une forme dépliée dès que la compression ayant crée la pliure est relâchée.

Le « coefficient de friction » peut être mesuré selon la norme ASTM D1894, par exemple.

Les valeurs du coefficient de friction mentionné ci-dessus permettent qu'il n'y ait pas d'adhérence des deux faces opposées de la paroi interne de la gaine, l'une contre l'autre pour ne créer aucune résistance au passage des gaz et matières dans la gaine d'une part, et d'autre part pour qu'en cas de pliure la gaine ne se bouche pas en se refermant sur elle-même.

Plus particulièrement encore, ladite gaine est réalisée en matériau polymérique biocompatible à base de silicone comprenant au moins les composés suivants :
- un élastomère de grade ou qualité « LSR » (« Liquid Silicon Rubber ») dans une proportion pondérale de 75 à 95%,
- un élastomère de grade ou qualité « RTV » dans une proportion pondérale de 2,5 à 12.5 %,
- un élastomère de grade ou qualité « gel » dans une proportion pondérale de 2,5 à 12.5 %.

Cette combinaison d'élastomères de différents grades (« LSR »+ « RTV »+ « gel ») est avantageuse car :
- un élastomère de grade ou qualité LSR apporte la résistance à la déchirure.
- un élastomère de grade ou qualité RTV apporte des propriétés d'élasticité radiale et longitudinale, et
- un élastomère de grade ou qualité gel apporte un coefficient d'adhérence réduit (non collabilité).

Dans un mode préféré de réalisation, ledit élément d'ancrage est une prothèse entérale du type stent recouvert sur au moins sa face externe d'un revêtement en matériau synthétique élastomère de type silicone ou polyuréthane, ledit revêtement élastomère étant plus souple que le matériau élastomère constitutif de ladite gaine, la liaison entre ladite gaine et ledit élément d'ancrage se faisant par recouvrement dudit élément d'ancrage par ladite gaine sur une partie seulement de la longueur dudit élément d'ancrage comprenant au moins son extrémité avale profilée de diamètre diminuant progressivement.

Des prothèses entérales de type stent recouverts d'élastomère, notamment à base de silicone de ce type sont bien connues et disponibles dans le commerce.

Ainsi, l'élastomère recouvrant le stent présente de plus grandes déformabilités radiale et longitudinale que l'élastomère de la gaine, c'est-à-dire que l'élastomère du revêtement du stent est constitué le cas échéant d'un mélange de différents types de silicone, présentant une teneur pondérale en grade RTV supérieur à celui de l'élastomère de la gaine, tout en restant dans la fourchette mentionnée ci-dessus de 2,5 à 12,5%.

Comme explicité ci-après, ce mode de réalisation permet en exerçant une traction sur la gaine d'induire une réduction du diamètre et une élongation axiale du stent permettant de le désolidariser de l'intestin pour faciliter sa migration à travers l'anastomose lors de la libération dudit élément d'ancrage.

Avantageusement encore, on met en oeuvre un dit élément d'ancrage de type stent comportant une prolongation longiligne ou filiforme à son extrémité, appelée de façon connue « lasso » permettant d'effectuer de façon connue de l'homme du métier, éventuellement avec un outil tel qu'un coloscope, une traction sur l'extrémité dudit élément d'ancrage du type stent, afin d'en faciliter la libération et/ou la migration à travers la zone d'anastomose lorsque cela est souhaité de façon à éviter un blocage à ce niveau.

Avantageusement, ledit matériau polymérique de ladite gaine comprend des filaments radio opaque, notamment de sulfate de baryum, disposés dans la direction longitudinale du tube.

Ces filaments permettent de suivre la migration du tube de la gaine et contrôler son positionnement initial puis sa migration progressive lors de son élimination. D'autre part, le positionnement longitudinal de ces filaments confère à la gaine simultanément une résistance à l'allongement et réduit son élasticité longitudinale qui peut être excessive comme par rapport à celle de l'intestin comme explicité précédemment.

Avantageusement, la gaine est graduée dans le sens croissant en partant de son extrémité amont.

Plus particulièrement, l'introducteur peut être de façon connue constitué d'un tube guide semi rigide, du type cathéter muni à une de ses extrémités d'une poignée et dont le diamètre interne et la longueur permettent d'y maintenir logé ledit élément d'ancrage sous sa forme rétractée et ladite gaine, de préférence déployée longitudinalement.

Toutefois, de préférence, ledit dispositif comprend en outre un instrument dénommé introducteur comprenant :
- une enveloppe externe tubulaire apte à contenir et maintenir ledit élément d'ancrage comprimé en dite position rétractée à l'intérieur de l'extrémité distale da dite enveloppe externe, et suffisamment long pour contenir en outre ladite gaine, ladite enveloppe externe étant de préférence d'une longueur d'au moins 110 cm, de préférence au moins 150 cm, et
- des moyens pour acheminer l'extrémité distale dudit introducteur depuis l'orifice anal jusqu'au dit site d'ancrage dans l'intestin en amont de l'anastomose, et
- de préférence, des moyens pour dégager ledit élément d'ancrage par rapport à l'enveloppe externe, de préférence encore consistant en un tube butoir comportant une butée à son extrémité distale, en contact le cas échéant avec l'extrémité longitudinale dudit élément d'ancrage, ladite gaine en aval de l'élément d'ancrage entourant ledit tube butoir à l'intérieur de ladite enveloppe externe.

Le retrait de l'enveloppe externe, puis du tube butoir permet de déployer complètement la gaine en aval de l'élément d'ancrage sans qu'aucune manoeuvre supplémentaire du chirurgien ne soit nécessaire pour déployer la gaine.

De préférence encore, le dispositif selon l'invention comprend en outre un tube protecteur comprenant une partie de forme courbe selon la courbure de la concavité sacrée, de rigidité supérieure à celle de l'enveloppe externe de l'introducteur, de diamètre externe et longueur tels que ledit tube protecteur est apte à être introduit par l'orifice anal et s'étendre entre l'orifice anal et jusqu'en amont de ladite anastomose et un diamètre interne et une dite courbure, selon la courbure de la concavité sacrée, tels que ledit tube protecteur est apte à contenir ledit introducteur et à en permettre l'acheminement entre l'orifice anal et ladite anastomose, de préférence un tube protecteur de diamètre externe de 20 à 40 mm et de longueur de 10 à 25 cm.

La présente invention permet de mettre en oeuvre un procédé de traitement chirurgical à l'aide d'un dispositif chirurgical selon l'invention dans on réalise la protection temporaire d'une anastomose sur le gros intestin ou côlon, le rectum ou canal anal, afin de prévenir ou réduire les risques de fistule anastomotique, en effectuant les étapes successives suivantes dans lesquelles :
1) On introduit un dit dispositif chirurgical par voie anale et on l'achemine jusqu'à un site d'ancrage en amont de la zone d'anastomose, ledit élément d'ancrage étant maintenu en dite position radiale rétractée D'₁ et acheminé à l'aide d'un instrument dénommé introducteur, et ladite gaine élastomère étant d'une longueur au moins égale à la distance entre le site d'ancrage et l'orifice anal, et
2) On dégage l'introducteur par rapport audit élément d'ancrage une fois acheminé au niveau du dit site d'ancrage de manière à permettre ainsi que ledit élément d'ancrage adopte une dite position d'ancrage contre la paroi intestinale en dite position d'expansion radiale maximale.

De préférence, la distance entre ladite position d'ancrage et ladite anastomose est au moins égale à la distance parcourue en au moins trois jours de préférence au moins cinq jours par ledit élément d'ancrage en migration une fois libéré de ladite paroi intestinale par la reprise du transit intestinal.

Plus particulièrement, la distance entre l'anastomose et le site d'ancrage est d'au moins 50 cm, de préférence au moins 1 m.

Plus particulièrement encore, ledit élément d'ancrage reste ancré dans ladite position d'ancrage au moins trois jours de préférence au moins cinq jours après son ancrage en l'absence de reprise du transit intestinal.

Ainsi, dans la mesure où la phase de paralysie intestinale post opératoire dite iléus dure de 3 à 5 jours, il est ainsi possible de protéger l'anastomose de cette même période additionnée du temps que met ledit élément d'ancrage pour migrer après libération entre son site d'ancrage en amont de l'anastomose et le site de l'anastomose, étant entendu que ce temps de migration dépend de la distance à parcourir entre le site d'ancrage et l'anastomose. En pratique une distance de 50cm à 1m se traduit par un temps de migration de 3 à 6 jours, de sorte qu'au total l'anastomose sera protégée pendant une durée de 6 à 11 jours après la réalisation de l'anastomose.

De préférence, on introduit ledit introducteur en le poussant à l'intérieur d'un tube protecteur comprenant une partie de forme courbe selon la courbure de la concavité sacrée, semi rigide, de rigidité supérieure à celle de l'enveloppe externe de l'introducteur, de diamètre externe et longueur, tels que ledit tube protecteur est apte à être introduit par l'orifice anal et s'étendre entre l'orifice anal et jusqu'en amont de ladite anastomose, et un diamètre interne et une dite courbure, selon la courbure de la concavité sacrée, tels que ledit tube protecteur est apte à contenir ledit introducteur et à en permettre l'acheminement entre l'orifice anal et ladite anastomose, celui-ci s'étendant entre l'orifice anal et ladite anastomose, de préférence un tube protecteur de diamètre externe de 20 à 40 mm et de longueur de 10 à 25 cm.

De préférence encore, on récupère ledit élément d'ancrage en migration une fois libéré de ladite paroi intestinale par la reprise du transit intestinal, dans un tube protecteur comprenant une partie de forme courbe selon la courbure de la concavité sacrée semi rigide, de rigidité supérieure à celle de l'enveloppe externe de l'introducteur, de diamètre externe et longueur tels que ledit tube protecteur est apte à être introduit par l'orifice anal et s'étendre entre l'orifice anal et jusqu'en amont de ladite anastomose, et un diamètre interne et une dite courbure, selon la courbure de la concavité sacrée, tels que ledit tube protecteur est apte à contenir ledit introducteur et à en permettre l'acheminement entre l'orifice anal et ladite anastomose, celui-ci s'étendant entre l'orifice anal et ladite anastomose, de préférence un tube protecteur de diamètre externe de 20 à 40 mm et de longueur de 10 à 25 cm.

D'autres caractéristiques et avantages de la présente invention apparaîtront à la lumière de la description détaillée qui va suivre.

En référence aux figures 1 à 6 dans lesquelles :
- la figure 1 représente schématiquement un dispositif selon l'invention,
- la figure 2 représente une vue en coupe longitudinale d'un mode de réalisation d'un dispositif selon l'invention avec une partie terminale avale 2b de l'élément d'ancrage profilé de diamètre réduit par rapport à une partie courante cylindrique,
- la figure 3 représente une vue schématique du système digestif, avec une anastomose 5,
- la figure 4 représente une vue schématique du dispositif selon l'invention acheminé à son site d'implantation à travers un tube introducteur 4, l'élément d'ancrage 2 étant en position radiale rétractée au sortir de l'introducteur et la gaine 3 étant encore logée à l'intérieur de l'introducteur.
- la figure 5 représente le dispositif selon l'invention, en partie en position d'ancrage, l'élément d'ancrage étant maintenant en position d'expansion radiale maximale bloqué contre la paroi de l'intestin.
- la figure 6 représente schématiquement le dispositif selon la présente invention en position d'ancrage et de protection de l'anastomose, la gaine 3 étant déployée en aval de l'élément d'ancrage 2.
- les figures 7A et 7B représentent le dispositif selon l'invention en migration en phase péristaltique d'élimination (Figure 7A) et en fin d'élimination (figure 7B).
- la figure 8 représente un introducteur selon l'invention.

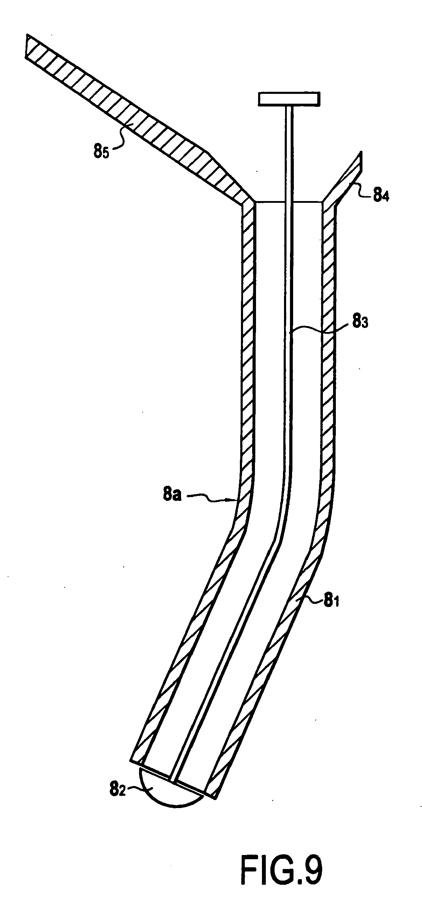

- La figure 9 représente un tube protecteur selon l'invention.
- La figure 10 représente un introducteur selon l'invention à travers le tube protecteur au niveau de la concavité sacrée.
- La figure 11 représente une vue schématique d'un mode de réalisation préféré d'un dispositif selon l'invention.

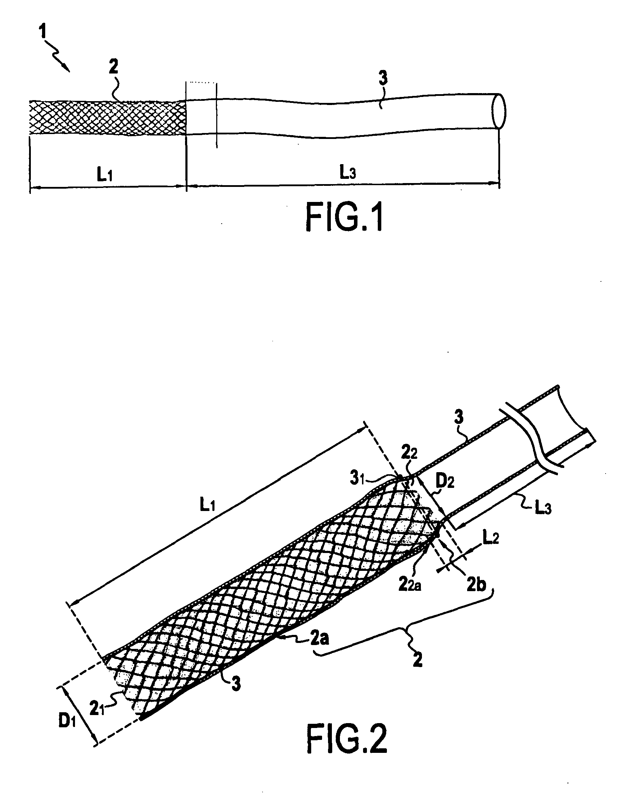

Sur la figure 1, on a représenté un dispositif chirurgical selon l'invention comprenant :
- un élément d'ancrage 2 constitué par une prothèse entérale en position d'expansion radiale maximale, de type décrit ci-après, et
- une gaine souple 3 en silicone solidarisée à l'extrémité avale de la prothèse entérale cylindrique, s'étendant sur une longueur L3 au moins deux fois la longueur de l'élément d'ancrage 2.

La prothèse 2 consiste en un dispositif connu sous le nom de « stent » réalisé notamment en nitinol dont les propriétés physiques sont connues de l'homme du métier. Il s'agit de petits tubes ayant des propriétés d'expansion radiale contrôlée en fonction de la température. Plus précisément, ils connaissent une expansion radiale progressive dès qu'ils sont mis à température supérieure à la température d'environ 20°C. Il s'agit d'un élément réalisé par maillage spiralé de fils de nitinol entièrement revêtu d'une ou plusieurs couches de silicone.

Sur la figure 2, on a représenté une variante préférée de réalisation de l'élément d'ancrage ou « stent » 2, dans laquelle ladite partie courante 2a de dite première paroi s'étend depuis l'extrémité longitudinale amont 2₁ de ladite première paroi jusqu'à une partie terminale avale profilée 2b de diamètre plus petit que celui de la partie courante cylindrique. Le diamètre D1 de la partie courante cylindrique en expansion radiale maximale est d'environ 32 mm. Le diamètre externe en expansion radiale maximale D2 de l'extrémité avale 2₂ de la première paroi est d'environ 24 mm. La longueur L1 de la partie courante cylindrique est d'environ 70 mm. Et, la longueur L2 de la partie terminale profilée 2b de la première paroi est d'environ 20 mm. Le diamètre de ladite partie terminale 2b diminue progressivement entre l'extrémité avale 2₂ de ladite partie courante 2a et ladite extrémité avale 2₂ de ladite première paroi.

Plus précisément, la réduction de diamètre de ladite partie terminale avale profilée 2b est obtenue en entourant celle-ci d'un anneau 3₁ en matériau élastomère de diamètre inférieur au dit diamètre d'expansion maximale radiale de ladite partie courante.

En position rétractée, cette prothèse 2 peut adopter un diamètre de 3 à 8 mm.

La gaine 3, comme l'anneau élastique 3₁ sont réalisés en matériau élastomère biocompatible à base de silicone ou de polyuréthane.

Dans un mode de réalisation représenté figure 2, la gaine à son extrémité amont recouvre l'élément d'ancrage 2 sur toute sa longueur, celui-ci étant noyé dans la masse de l'élastomère constituant la gaine, ce qui permet de constituer un revêtement de toute la surface externe dudit élément d'ancrage 2, tout en assurant la liaison entre la gaine et l'élément d'ancrage 2.

Ladite gaine présente au repos une longueur en aval dudit élément d'ancrage d'au moins 50 cm, de préférence au moins 1 m, et un diamètre D₂ de 26 à 33 mm, et une épaisseur de paroi de 0,1 à 0,5 mm.

Cette distance d'au moins 50 cm, de préférence 1 m permet d'envisager une implantation 7 de l'élément d'ancrage 2 à une distance en amont de l'anastomose 5 d'au moins 50 cm, respectivement au moins environ 1 m.

Pour la réalisation de la gaine 3, on utilise un mélange de différents types de silicone de type « LSR », « RTV » et « GEL », tels que décrits précédemment.

Le chirurgien réalise l'anastomose selon la technique habituelle, souvent au moyen d'une agrafeuse. Une fois l'anastomose réalisée, l'assistant introduit l'introducteur.

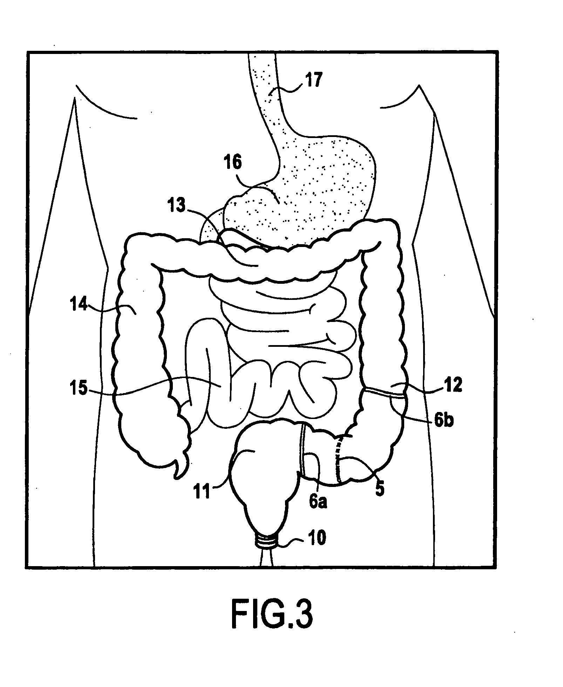

Sur la figure 3, on a représenté les différentes parties de l'intestin, à savoir :
- le rectum 11, le canal anal 10, le côlon gauche 12, le côlon transverse 13, le côlon droit 14, le petit intestin 15, l'estomac 16 et l'oesophage 17.

On a représenté en 6a et 6b les deux extrémités du segment d'intestin prélevé et en 5 la liaison réalisée entre ces deux extrémités constituant l'anastomose.

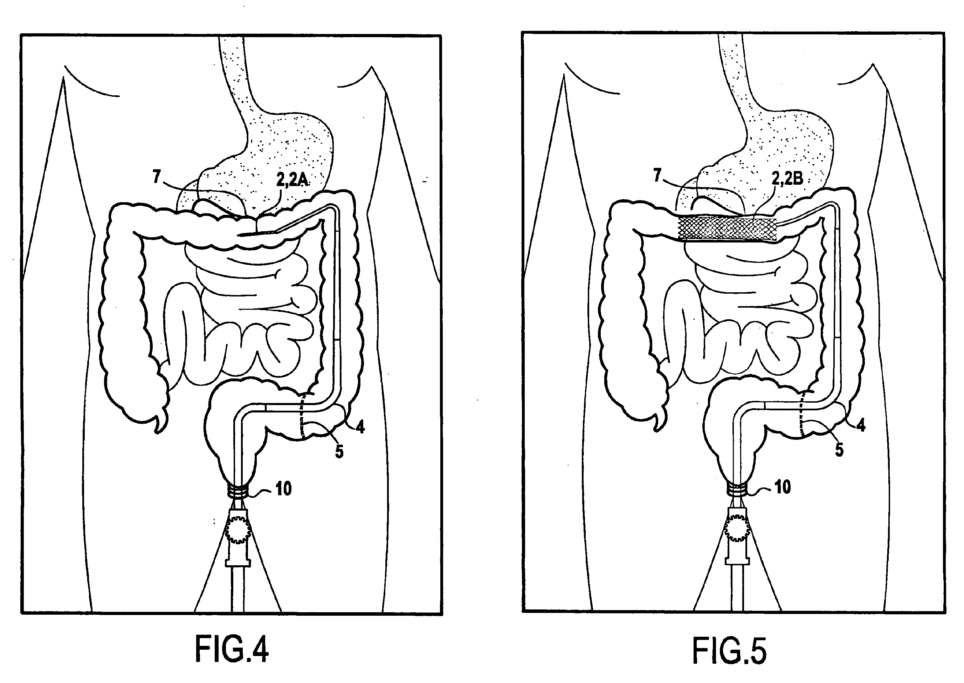

Sur la figure 4, on montre l'introduction du dispositif selon l'invention qui est introduit sous forme rétractée 2A par l'intermédiaire d'un introducteur 4 qui est constitué d'un tube en plastique semi rigide apte à être déformé de diamètre 3 à 8 mm, de longueur de 70 à 220 cm, au sein duquel l'élément d'ancrage est introduit sous forme rétractée, ladite gaine étant positionnée en aval de l'élément d'ancrage à l'intérieur du tube guide de l'introducteur. Une fois que le tube guide de l'introducteur est arrivé au site d'implantation 7, par exemple à environ 1 m en amont de l'anastomose 5, l'élément d'ancrage peut être sorti de l'extrémité de l'introducteur et il prend une position expansée 2B conformément à la figure 5. Il y a lieu de noter que le temps d'introduction et d'acheminement de l'introducteur de l'élément d'ancrage jusqu'au site d'implantation 7, est en pratique inférieur au temps à partir duquel ledit élément d'ancrage subit une expansion radiale de part l'augmentation de la température qu'il connaît à l'intérieur du corps.

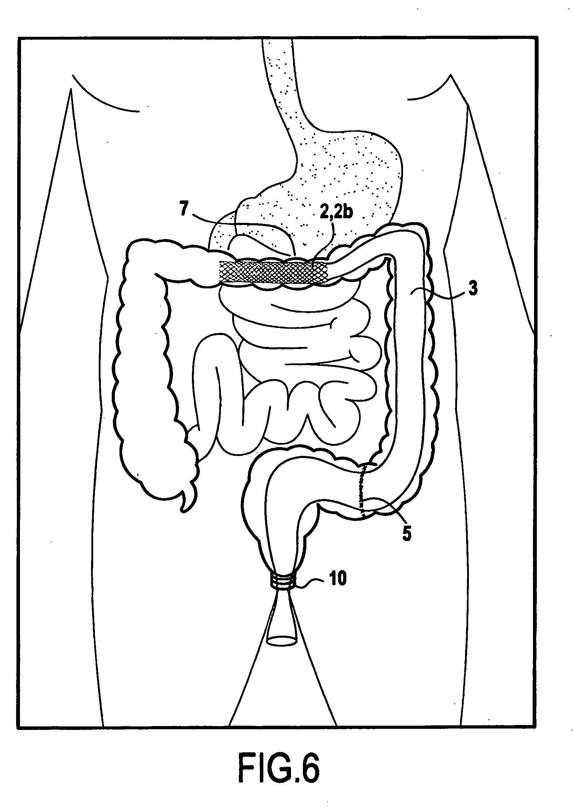

Sur la figure 6, on a représenté le dispositif complet avec la gaine 3 déployée après retrait de l'introducteur 4. La gaine 3 s'étend depuis le site d'implantation 7 jusqu'à l'orifice anal 10.

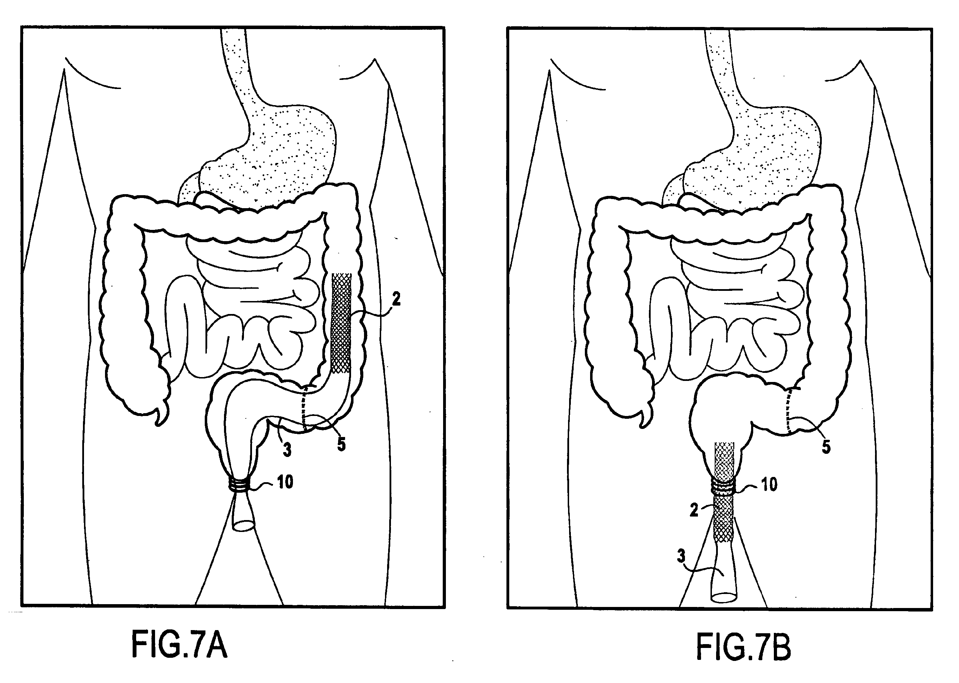

Dans sa forme initiale, fermé et logé dans l'introducteur, le stent présente un diamètre très réduit notamment de 3 à 8 mm. On traverse l'anastomose 5, puis on pénètre l'intestin 12 d'amont. Le chirurgien apprécie la progression de l'introducteur 4 et son bon emplacement par la palpation de l'introducteur à travers les parois de l'intestin et en visualisant le stent pendant son expansion. Une fois libérée dans la lumière de l'intestin, le stent retrouve progressivement son diamètre définitif. Il peut être temporairement maintenu en place par la main du chirurgien, qui pince le stent à travers les parois de l'intestin. L'introducteur est ensuite retiré. La gaine 3 est dépliée spontanément et progressivement au retrait de l'introducteur 4. Ce dernier retraverse l'anastomose puis l'orifice anal 10 dans le sens antégrade, libérant totalement la gaine 3. Après un délai moyen de 4 à 6 jours, et sous l'effet des contractions intestinales, l'unité stent-manchon migre progressivement vers l'orifice anal 10 depuis le site d'ancrage en amont, lequel est suffisamment en amont de préférence à au moins un mètre de longueur d'intestin de l'anastomose pour que le stent atteigne l'orifice anal 5 ou 6 jours plus tard seulement après reprise du transit intestinal, puis, le dispositif est éliminé avec la matière fécale, comme représenté sur les figures 7A et 7B.

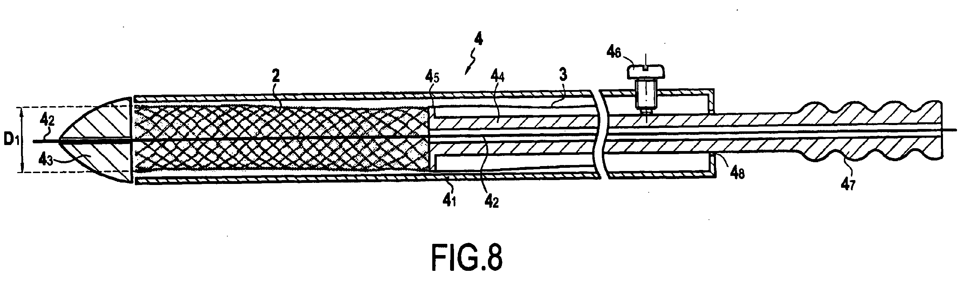

Sur la figure 8, on a représenté un introducteur 4 comprenant :
- une enveloppe externe tubulaire 4₁ droite, en matériau semi rigide de manière à pouvoir être courbée pour suivre les contours de l'intestin lorsqu'on achemine l'introducteur dans l'intestin depuis l'orifice anal. L'enveloppe externe présente un diamètre externe de 20 à 40 mm inférieur à celui de la paroi de l'intestin au niveau de l'anastomose et un diamètre interne et une longueur aptes à contenir ledit élément d'ancrage 2 comprimé en position rétractée, et ladite gaine dépliée derrière l'élément d'ancrage, soit en pratique une longueur de 100 à 150 mm.
- un tube butoir 4₄ disposé axialement à l'intérieur de ladite enveloppe et dont l'extrémité distale comporte une butée 4₅, qui vient buter contre ledit élément d'ancrage, lui-même bloqué par expansion radiale à l'extrémité distale de l'enveloppe externe 4₁. Le tube butoir présente un diamètre légèrement inférieur au diamètre interne de l'enveloppe externe de façon à contenir la gaine 3 fixée à l'extrémité avale de l'élément d'ancrage, ladite gaine entourant ledit tube butoir 4₁. Au retrait de l'enveloppe externe, le tube butoir retient et donc évite le retrait de l'élément d'ancrage avec l'enveloppe et force l'élément d'ancrage à sortir et se déployer radialement contre la paroi de l'intestin.
- un fil guide 4₂ souple inséré dans l'axe du tube butoir et l'axe dudit élément d'ancrage contenu par l'enveloppe externe en position rétractée. L'extrémité distale du fil guide est en forme de « J » pour le rendre atraumatique lors de son introduction. Il peut être en métal ou en matériau synthétique.
- un cône atraumatique 4₃ perforé axialement pour laisser passer le fil guide 4₂. Le cône 4₃ de diamètre supérieur au diamètre interne de l'enveloppe externe est disposé en aval de l'enveloppe externe de façon à faciliter le cheminement de l'introducteur dans l'intestin.

La mise en place de la prothèse se fait de la manière suivante.
1) - On introduit le fil guide dans l'intestin jusqu'au site d'ancrage de l'élément d'ancrage.
2) - On introduit l'extrémité proximale à l'extérieure de l'orifice anal dans la perforation du cône atraumatique puis dans le canal axial de l'élément d'ancrage puis du tube butoir l'axe de l'introducteur ;
3) - On pousse l'extrémité distale de l'enveloppe externe de l'introducteur le long du fil guide dans l'intestin, ce qui pousse le cône atraumatique jusqu'en amont de l'anastomose pour atteindre le site d'ancrage.
4) - On retire le fil guide 4₂.
5) - On retire partiellement l'enveloppe externe 4₁ en maintenant le tube butoir 4₄ de manière à libérer ledit élément d'ancrage.
6) - On retire complètement l'enveloppe externe, le tube butoir et le cône atraumatique 4₃.

Selon la présente invention, le dispositif est mis en oeuvre à titre préventif sur une anastomose sans fistule. Néanmoins, la mise en place du dispositif 1 avec l'introducteur 4, comporte des manoeuvres lors de son introduction qui présentent un danger potentiel pour l'anastomose car elles pourraient la blesser. Pour éviter cet écueil, on met en oeuvre avantageusement un tube semi-rigide dénommé tube protecteur 8.

Le tube protecteur 8 sert à protéger momentanément l'anastomose lors de la mise en place du dispositif c'est-à-dire lors de l'introduction de l'introducteur 4. Le tube protecteur 8 est donc mis en place avant même la mise en place de l'introducteur 4 et sa manipulation. Une fois l'anastomose réalisée, on introduit d'abord le tube protecteur.

Ce tube protecteur 8 est plus rigide que l'introducteur 4 et protège la paroi intestinale, de manière à ce que l'introducteur 4 ne déforme pas la paroi intestinale lors de son introduction.

La forme courbe du tube protecteur 8 lui permet d'épouser les courbures de l'intestin. Il s'agit d'un tube présentant une partie centrale courbe 8a similaire à la courbure de la colonne vertébrale à son extrémité inférieure appelée par l'homme de l'art courbure de concavité sacrée. On retrouve des courbures de ce type dans les dispositifs d'agrafeuses. Ce tube 8 est ouvert à ses deux extrémités. L'extrémité proximale du tube protecteur 8 est évasée 8₄, ce qui lui permet d'épouser la forme anatomique de la marge anale, à l'extérieur de l'orifice anal externe. Cette même extrémité comporte à sa face externe une tige droite 8₅ légèrement inclinée qui sert à saisir le tube. L'extrémité distale du tube 8 est non évasée pour être introduite dans l'orifice anale, puis acheminée jusqu"en amont de l'anastomose 5. Pour ce faire, cette extrémité non évasée du tube 8 peut être de préférence obturée de façon réversible par un cône atraumatique 8₂ monté sur une tige 8₃ située dans la lumière du tube protecteur. Ce cône atraumatique 8₂ évite de blesser l'intestin lors de l'introduction du tube protecteur 8.

Le tube protecteur 8 aura une longueur correspondant au moins à la distance entre l'orifice anal et l'anastomose, soit en pratique au moins de 5 à 25 cm et un diamètre extérieur inférieur ou égal à celui de l'intestin au niveau de l'anastomose, en pratique le diamètre de l'agrafeuse utilisée pour l'anastomose, soit de 10 à 40 mm, de préférence 20 à 30 mm. Et, son diamètre interne est au moins celui de l'introducteur pour permettre le passage de l'introducteur 4.

En pratique, il existe 3 types d'anastomoses : colo-colique (côlon avec côlon), colo-rectale (côlon avec rectum), colo-anale (côlon avec canal anal). Selon le type d'anastomose réalisée, la zone de réunion entre les 2 extrémités d'intestin se trouve plus ou moins distante par rapport à l'orifice anal (qui à cette occasion sert comme point de repère). Ainsi en partant de l'orifice anal vers l'amont, l'anastomose colo-anale sera située de 1 à 3 cm de l'orifice anal, l'anastomose colo-rectale sera située de 4 à 10 cm de l'orifice anal et l'anastomose colo-colique sera située de 11 à 20 cm de l'orifice anal. C'est la raison pour laquelle la longueur du tube 8 est jusqu'à 25 cm, afin de pouvoir atteindre une anastomose colo-colique, celle située le plus loin de l'orifice anal. Pour les anastomoses situées plus près de l'orifice anal, le tube peut ne pas être introduit sur toute sa longueur. Le positionnement exact du tube 8 se fait dans tous les cas sous le contrôle visuel de l'opérateur.

Une fois l'extrémité distale du tube protecteur acheminé en amont de l'anastomose quelques cm en amont de l'anastomose, le cône atraumatique est retiré pour libérer la lumière du tube protecteur. On introduit alors l'introducteur 4 dans la lumière du tube protecteur. Ainsi le tube protecteur 8 de par sa semi rigidité protège l'anastomose 5 contre les frottements de l'introducteur 4. Plus l'introducteur remonte dans l'intestin d'amont plus les frottements exercés contre les parois de l'intestin sont importants et plus la pression exercée par l'opérateur sur l'introducteur devient importante. La lumière du tube digestif a un coefficient de résistance non négligeable de par ses parois internes et les contractions de l'intestin même. Ces remarques s'appliquent aussi au retrait de l'introducteur 4.

En pratique, une fois l'anastomose réalisée, on saisit le tube 8 avec son cône atraumatique 8₂, par la tige 8₃, on introduit l'ensemble dans l'intestin, à travers l'orifice anal. Le tube protecteur 8 est poussé à travers l'anastomose 5 dans l'intestin d'amont 12. L'extrémité distale du tube 8 est positionnée en 8 et la tige 8₃ viennent au contact de la marge anale et sont maintenues en place par la main de l'opérateur. Le cône atraumatique 8₂ de diamètre légèrement inférieur à celui du tube 8 est retiré. Puis, on introduit l'introducteur 4 à travers l'orifice évasé 8₄ du tube protecteur pour le pousser à travers le tube protecteur 8. Les étapes suivantes se déroulent comme précédemment décrit concernant la mise en oeuvre de l'introducteur 4. Au total l'ensemble des manoeuvres réalisées lors de la manipulation de l'introducteur 4 se fait à travers le tube protecteur 8, qui est une barrière de protection pour l'anastomose et évite de la blesser. Après le retrait complet de l'introducteur 4 le tube protecteur est retiré à son tour.

La terminaison profilée 2b du stent 2 est de nature à faciliter sa progression dans la lumière intestinale. Néanmoins, la région d'anastomose présente souvent une sténose au niveau de la jonction entre les deux extrémités d'intestin et a donc un diamètre plus réduit que l'intestin situé de part et d'autre. Ceci est dû à la cicatrisation de l'anastomose. Cette sténose peut être une gêne potentielle à la progression du stent 2 à travers l'anastomose 5 et à l'extrême, source d'enclavement du stent dans cette zone. On peut toujours faciliter la migration du stent par des manoeuvres de traction exercées sur la partie proximale de la gaine 3, mais ce n'est parfois pas suffisant. Dans ce cas on peut s'aider d'un tube protecteur 8, qui facilite le passage du stent ou qui permet dans le cas extrême de désenclaver le stent.

Il s'agit d'un tube cylindrique semi-rigide qui a la courbure de la concavité sacrée et celle de l'intestin, comme le tube protecteur 8 décrit ci-dessus, mais s'étendant depuis l'orifice anal jusqu'en amont de l'anastomose 5. L'une de ses extrémités dite proximale est évasée pour s'adapter à la conformation de la marge anale. Mais, son diamètre interne est au moins supérieur à celui de l'élément d'ancrage ou stent en position radiale rétractée. Sa longueur est identique à celle du tube protecteur pour la mise en place de l'introducteur 4.

La manoeuvre consiste à repérer la zone d'anastomose sous contrôle de radioscopie. Cette zone est facilement retrouvée par la présence des agrafes qui ont servi à la réalisation de l'anastomose, et par la présence du stent qui porte des marques radio-opaques.

Lorsque l'extrémité avale de la gaine arrive juste en aval de l'anastomose 5, l'extrémité avale de la gaine 3 est introduite dans la lumière du tube 8 à travers son extrémité distale. La gaine est extériorisée à travers l'extrémité proximale du tube protecteur. Le tube protecteur 8 est glissé progressivement le long de la gaine 3, qui lui sert de fil guide. Il pénètre le canal anal puis dans l'intestin. Il est poussé par l'opérateur pour venir au contact de l'extrémité avale du stent pou y être immobilisé. On tire progressivement sur l'extrémité avale de la gaine. L'extrémité amont de la gaine attachée au stent pénètre dans la lumière distale du tube. On continue la traction sur l'extrémité avale de la gaine. L'extrémité avale du stent suit l'extrémité amont de la gaine. Les propriétés de rétraction du stent lui permettent d'adopter le diamètre interne du tube et de pénétrer dans la lumière distale du tube dont le diamètre interne est au moins celui de l'introducteur. La traction continue sur la gaine avale permet au stent de pénétrer entièrement dans le tube 8. Puis l'ensemble du tube 8 contenant le stent et la gaine est retiré à travers le canal anal.

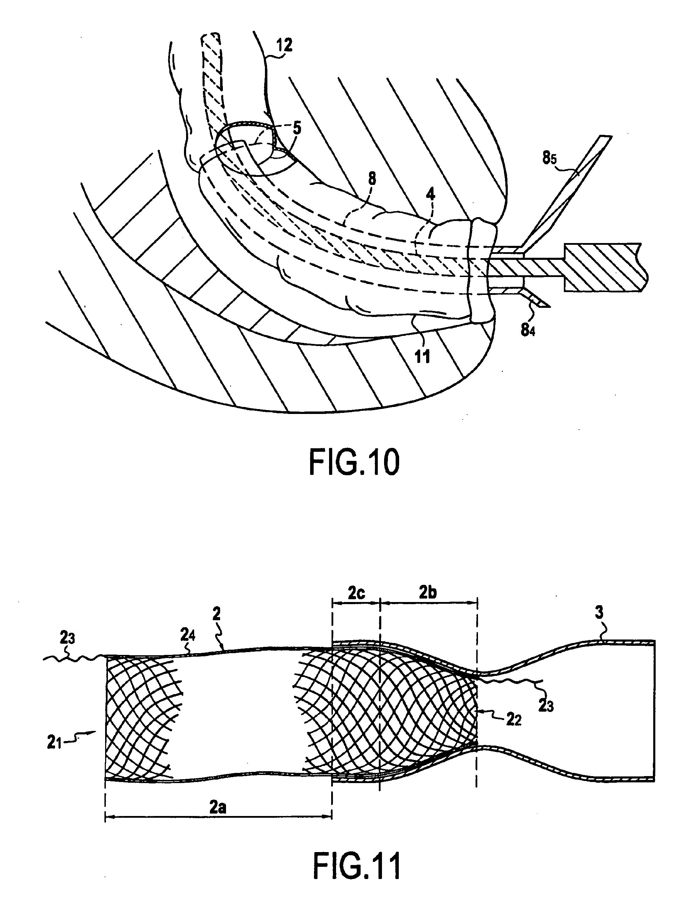

Sur la figure 11, on a représenté une version préférée d'un dispositif selon l'invention dans lequel la gaine 3 recouvre partiellement seulement le stent 2, de manière à recouvrir toute son extrémité avale conique, la liaison entre la gaine 3 et le stent 2 débutant en amont de l'extrémité conique approximativement sur une zone 2c de 3 à 30 mm, de préférence 5 à 15 mm de longueur en amont de l'extrémité amont de la zone profilée 2b.

Le stent 2, qui est réalisé en un maillage de fils spiralés est recouvert d'un revêtement silicone 24 plus fin que ladite gaine, conférant au dit stent une plus grand déformabilité radiale et longitudinale que ladite gaine. Ainsi, en cas de blocage du stent en amont de l'anastomose, empêchant son relargage et/ou sa migration à travers l'anastomose, on peut exercer manuellement une traction sur l'extrémité proximale de ladite gaine, déformant ainsi par réduction de son diamètre et allongement le reste du stent pour faciliter son passage à travers l'anastomose.

De préférence encore, le stent 2 possède à chaque extrémité, une extension filiforme connue sous le nom de « lasso » long de 10 mm environ, de préférence réalisée dans le même matériau que le stent. Ainsi, une traction sur le lasso, à l'extrémité aval du stent permet de façon connue, au moyen d'un instrument de type coloscope ou rectoscope existant dans le commerce de faciliter la réduction de diamètre et l'allongement axiale du stent.

D'autre part, toujours de façon connue une traction sur le « lasso » en amont du stent permet, en cas de blocage du stent, d'effectuer un retournement vers l'intérieur du stent, en tirant son extrémité amont 2₁ d'amont en aval à l'intérieur de lui-même, de façon à le débloquer de la paroi intestinale dans laquelle il peut être le cas échéant incarcéré.

De façon connue, l'extrémité filiforme 2₃ du stent, appelée « lasso » s'étend depuis la périphérie constitutive de l'extrémité aval 2₂ ou amont 2₁ dudit stent.

Dans un autre mode de réalisation, en plus ou à la place de l'extrémité aval filiforme appelée « lasso » décrite ci-dessus, on attache à l'extrémité proximale (aval) du stent, en faisant le tour de sa périphérie en se faufilant entre les mailles, un brin souple de même caractéristique que le lasso. Ainsi, une traction sur ce brin entraîne la fermeture presque complète de l'extrémité aval proximale du stent où commence la gaine. Ce brin s'étend à l'intérieur de ladite gaine jusqu'à son extrémité proximale située à l'anus, de sorte que l'accès au dit brin ne nécessite pas obligatoirement l'usage d'un coloscope ou rectoscope. En cas d'incarcération du stent la fermeture de l'extrémité proximale du stent à l'aide de traction sur le brin aide à faciliter le passage du stent à travers l'anastomose.

## Revendications

1. Dispositif chirurgical (1) apte à réaliser la protection temporaire d'une anastomose (5) sur le côlon (12,13,14), le rectum (11) ou le canal anal(10) comprenant :
a) un élément longitudinal creux semi-rigide dit élément d'ancrage temporaire (2) dont la paroi dite première paroi, définit une surface de révolution autour d'un axe longitudinal comprenant une partie courante (2a) sensiblement cylindrique à section sensiblement circulaire, ladite première paroi présentant un diamètre externe que l'on peut faire varier de façon contrôlée entre :
- un diamètre externe réduit en position radiale rétractée de ladite première paroi d'au plus 10mm, et
- un diamètre externe maximal (D1) en position d'expansion radiale maximale de la partie courante de ladite première paroi, de 20 à 40 mm, de préférence de 25 à 33 mm, et
b) une gaine (3) à paroi tubulaire souple fixée audit élément d'ancrage (2) contre, de préférence autour de ladite première paroi,
**caractérisé en ce que** :
1) ladite première paroi dudit élément d'ancrage temporaire (2) comprend une dite partie courante (2a) de plus grand diamètre, de longueur (L1) d'au moins 50 mm, de préférence de 70 à 150 mm, et
2) ladite gaine (3) présente au repos une longueur (L3) en aval dudit élément d'ancrage (2) d'au moins 50 cm, de préférence au moins 1m, et un diamètre externe de 20 à 40 mm, de préférence de 25 à 33mm, et ladite gaine est en matériau élastomère biocompatible d'épaisseur de paroi de 0,05 à 1mm.

2. Dispositif selon la revendication 1 **caractérisé en ce que** ladite partie courante (2a) de dite première paroi s'étend depuis l'extrémité longitudinale amont (2₁) de ladite première paroi jusqu'à une partie terminale avale profilée (2b) de diamètre plus petit que celui de la partie courante cylindrique, le diamètre externe en expansion radiale maximale (D2) de l'extrémité avale (2₂) de ladite première paroi étant de 20 à 35 mm , et la longueur (L2) de ladite partie terminale profilée (2b) de la première paroi étant de 10 à 30 mm , de préférence de 15 à 25 mm, de préférence le diamètre de ladite partie terminale (2b) diminuant progressivement entre ladite partie courante et ladite extrémité avale de ladite première paroi.

3. Dispositif selon la revendication 2, **caractérisé en ce que** la réduction de diamètre de ladite partie terminale avale profilée (2b) est obtenue en entourant celle-ci d'un anneau (3₁) en matériau élastomère de diamètre inférieur au dit diamètre d'expansion maximale radiale (D₁) de ladite partie courante.

4. Dispositif selon la revendication 1 à 3 **caractérisé en ce que**
- ledit élément d'ancrage(2) est réalisé en un matériau lui conférant des propriétés d'élasticité radiale de sorte qu'il peut être comprimé radialement en dite position rétractée et adopter une dite position d'expansion radiale maximale (D₁) après relâchement de la compression radiale, et
- ladite gaine (3) est réalisée en matériau à base d'élastomère polymérique biocompatible du type silicone ou polyuréthane d'épaisseur de paroi de 0,1 à 0,5 mm et présentant des propriétés d'élasticité radiale et longitudinale, des propriétés de mémoire de forme et des propriétés de non collabilité.

5. Dispositif selon la revendication 4 **caractérisé en ce que** ledit élément d'ancrage (2) temporaire est une prothèse entérale de type stent dont ladite première paroi est formée par un maillage de fils spiralés métalliques ou élastomères.

6. Dispositif selon l'une des revendications 1 à 5 **caractérisé en ce que** la liaison entre ladite gaine et ledit élément d'ancrage se fait par recouvrement dudit élément d'ancrage par ladite gaine, au moins sur la surface externe de l'extrémité avale dudit élément d'ancrage.

7. Dispositif selon l'une des revendications 1 à 6 **caractérisé en ce que** ladite gaine est réalisée en matériau polymérique biocompatible à base d'élastomère présentant les propriétés suivantes :
- une dureté de 5 à 95 Shore A, de préférence de 50 à 70 Shore A,
- un degré de déformation rémanente à la compression de 10% à 35%, de préférence de 17% à 23 %,
- un coefficient de friction de 0,30 à 0,90, de préférence de 0,35 à 0,45.

8. Dispositif selon l'une des revendications 6 ou 7, **caractérisé en ce que** ledit élément d'ancrage (2) est une prothèse entérale du type stent recouvert sur au moins sa face externe d'un revêtement (24) en matériau synthétique élastomère de type silicone ou polyuréthane, ledit revêtement élastomère étant plus souple que le matériau élastomère constitutif de ladite gaine, la liaison entre ladite gaine et ledit élément d'ancrage se faisant par recouvrement dudit élément d'ancrage par ladite gaine sur une partie seulement (2b,2c) de la longueur dudit élément d'ancrage comprenant au moins son extrémité avale profilée (2b) de diamètre diminuant progressivement.

9. Dispositif selon l'une des revendications 1 à 8 **caractérisé en ce que** ledit dispositif comprend en outre un instrument dénommé introducteur (4) comprenant :
- une enveloppe externe tubulaire (4₁) apte à contenir et maintenir ledit élément d'ancrage comprimé en dite position rétractée à l'intérieur de l'extrémité distale da dite enveloppe externe, et suffisamment long pour contenir en outre ladite gaine (3), ladite enveloppe externe étant de préférence d'une longueur d'au moins 110 cm, de préférence au moins 150 cm, et
- des moyens (4₂,4₃) pour acheminer l'extrémité distale dudit introducteur depuis l'orifice anal (10) jusqu'au dit site d'ancrage dans l'intestin en amont de l'anastomose (5), et
- de préférence, des moyens (4₄,4₅) pour dégager ledit élément d'ancrage par rapport à l'enveloppe externe (4₁), de préférence encore consistant en un tube butoir (4₄) comportant une butée (4₅) à son extrémité distale en contact le cas échéant avec l'extrémité longitudinale dudit élément d'ancrage, ladite gaine en aval de l'élément d'ancrage entourant ledit tube butoir (4₄) à l'intérieur de ladite enveloppe externe.

10. Dispositif selon l'une des revendications 1 à 9 **caractérisé en ce qu'**il comprend en outre un tube protecteur (8) comprenant une partie (8a) de forme courbe selon la courbure de la concavité sacrée, de rigidité supérieure à celle de l'enveloppe externe de l'introducteur (4), de diamètre externe et longueur tels que ledit tube protecteur est apte à être introduit par l'orifice anal et s'étendre entre l'orifice anal et jusqu'en amont de ladite anastomose, et un diamètre interne et une dite courbure, selon la courbure de la concavité sacrée, tel que ledit tube protecteur est apte à contenir ledit introducteur et à en permettre l'acheminement entre l'orifice anal et ladite anastomose, de préférence un tube protecteur de diamètre externe de 20 à 40mm et de longueur de 10 à 25 cm.

## Patentansprüche

1. Chirurgische Vorrichtung (1), die geeignet ist, den vorübergehenden Schutz einer Anastomose (5) am Dickdarm (12, 13, 14), am Rektum (11) oder am Afterkanal (10) herzustellen, umfassend:
a) ein halbstarres, hohles, längliches Element, sogenanntes temporäres Verankerungselement (2), dessen Wand, sogenannte erste Wand, eine Rotationsfläche um eine Längsachse definiert, mit einem im wesentlichen zylindrischen durchgehenden Teil (2a) mit im wesentlichen kreisförmigem Querschnitt, wobei die erste Wand einen Außendurchmesser aufweist, der kontrolliert verändert werden kann zwischen:
- einem reduzierten Außendurchmesser von höchstens 10 mm in zusammengezogener radialer Position der ersten Wand und
- einem maximalen Außendurchmesser (D1) von 20 bis 40 mm, vorzugsweise von 25 bis 33 mm, in maximaler radialer Expansionsposition des durchgehenden Teils der ersten Wand, sowie
b) eine Hülle (3) mit flexibler röhrenförmiger Wand, die an dem Verankerungselement (2) an der vorzugsweise um die erste(n) Wand befestigt ist,
**dadurch gekennzeichnet, daß**:
1) die erste Wand des temporären Verankerungselements (2) einen durchgehenden Teil (2a) mit größerem Durchmesser, mit einer Länge (L1) von wenigstens 50 mm, vorzugsweise von 70 bis 150 mm umfaßt und
2) die Hülle (3) in Ruhe eine Länge (L3) stromabwärts des Verankerungselements (2) von wenigstens 50 cm, vorzugsweise wenigstens 1 m sowie einen Außendurchmesser von 20 bis 40 mm, vorzugsweise von 25 bis 33 mm aufweist und die Hülle aus einem biokompatiblen Elastomermaterial mit einer Wanddicke von 0,05 bis 1 mm besteht.

2. Vorrichtung nach Anspruch 1, **dadurch gekennzeichnet, daß** der durchgehende Teil (2a) der ersten Wand sich von dem stromaufwärtigen Längsende (2₁) der ersten Wand bis zu einem stromabwärtigen profilierten terminalen Teil (2b), dessen Durchmesser kleiner als der des zylindrischen durchgehenden Teils ist, erstreckt, wobei der Außendurchmesser in maximaler radialer Expansion (D2) des stromabwärtigen Endes (2₂) der ersten Wand 20 bis 35 mm beträgt und wobei die Länge (L2) des profilierten terminalen Teils (2b) der ersten Wand 10 bis 30 mm, vorzugsweise 15 bis 25 mm beträgt, wobei vorzugsweise der Durchmesser des terminalen Teils (2b) zwischen dem durchgehenden Teil und dem stromabwärtigen Ende der ersten Wand schrittweise abnimmt.

3. Vorrichtung nach Anspruch 2, **dadurch gekennzeichnet, daß** die Durchmesserverringerung des stromabwärtigen profilierten terminalen Teils (2b) dadurch erreicht wird, daß dieser von einem Ring (3₁) aus Elastomermaterial, dessen Durchmesser geringer als der maximale radiale Expansionsdurchmesser (D₁) des durchgehenden Teils ist, umschlossen wird.

4. Vorrichtung nach Anspruch 1 bis 3, **dadurch gekennzeichnet, daß**
- das Verankerungselement (2) aus einem Material gefertigt ist, das ihm Eigenschaften radialer Elastizität verleiht, so daß es in die zusammengezogene Position radial komprimiert werden kann und nach dem Lockern der radialen Kompression eine maximale radiale Expansionsposition (D₁) einnehmen kann, und
- die Hülle (3) aus einem Material auf der Basis eines biokompatiblen Polymer-Elastomers vom Typ Silikon oder Polyurethan mit einer Wanddicke von 0,1 bis 0,5 mm, das Radial- und Längselastizitätseigenschaften, Formgedächtniseigenschaften sowie Eigenschaften eines Nicht-Zusammenklebens aufweist, gefertigt ist.

5. Vorrichtung nach Anspruch 4, **dadurch gekennzeichnet, daß** das temporäre Verankerungselement (2) eine enterale Prothese vom Typ Stent ist, dessen erste Wand von einem Geflecht aus spiralförmigen Metall- oder Elastomerfäden gebildet ist.

6. Vorrichtung nach einem der Ansprüche 1 bis 5, **dadurch gekennzeichnet, daß** die Verbindung zwischen der Hülle und dem Verankerungselement durch Bedecken des Verankerungselements mit der Hülle, wenigstens auf der Außenfläche des stromabwärtigen Endes des Verankerungselements erfolgt.

7. Vorrichtung nach einem der Ansprüche 1 bis 6, **dadurch gekennzeichnet, daß** die Hülle aus einem biokompatiblen Polymermaterial auf Elastomerbasis gefertigt ist, das die folgenden Eigenschaften aufweist:
- eine Härte von 5 und 95 Shore A, vorzugsweise von 50 bis 70 Shore A,
- einen Grad bleibender Druckverformung von 10 % bis 35 %, vorzugsweise von 17 % bis 23 %,
- einen Reibungskoeffizienten von 0,30 bis 0,90, vorzugsweise von 0,35 bis 0,45.

8. Vorrichtung nach einem der Ansprüche 6 oder 7, **dadurch gekennzeichnet, daß** das Verankerungselement (2) eine enterale Prothese vom Typ Stent ist, der an wenigstens seiner Außenseite mit einer Beschichtung (24) aus synthetischem Elastomermaterial vom Typ Silikon oder Polyurethan überzogen ist, wobei die Elastomerbeschichtung flexibler ist als das die Hülle bildende Elastomermaterial, wobei die Verbindung zwischen der Hülle und dem Verankerungselement durch Bedecken des Verankerungselements mit der Hülle über lediglich einen Teil (2b, 2c) der Länge des Verankerungselements erfolgt, der wenigstens dessen stromabwärtiges profiliertes Ende (2b) mit schrittweise abnehmendem Durchmesser umfaßt.

9. Vorrichtung nach einem der Ansprüche 1 bis 8, **dadurch gekennzeichnet, daß** die Vorrichtung ferner ein als Introduktor (4) bezeichnetes Instrument aufweist, welches umfaßt:
- eine röhrenförmige Außenhülle (4₁), die geeignet ist, das in die zusammengezogene Position komprimierte Verankerungselement innerhalb des distalen Endes der Außenhülle zu enthalten und zu halten, und die lang genug ist, um ferner die Hülle (3) zu enthalten, wobei die Außenhülle vorzugsweise eine Länge von wenigstens 110 cm, vorzugsweise wenigstens 150 cm aufweist, und
- Mittel (4₂, 4₃), um das distale Ende des Introduktors von der Analöffnung (10) bis zu der Verankerungsstelle im Darm stromaufwärts der Anastomose (5) zu führen, und
- vorzugsweise Mittel (4₄, 4₅), um das Verankerungselement gegenüber der Außenhülle (4₁) zu lösen, die weiterhin vorzugsweise aus einem Anschlagrohr (4₄), bestehen, das an seinem distalen Ende einen Anschlag (4₅) umfaßt, der gegebenenfalls mit dem Längsende des Verankerungselements in Kontakt ist, wobei die Hülle stromabwärts des Verankerungselements das Anschlagrohr (4₄) innerhalb der Außenhülle umschließt.

10. Vorrichtung nach einem der Ansprüche 1 bis 9, **dadurch gekennzeichnet, daß** sie ferner ein Schutzrohr (8) umfaßt, mit einem Teil (8a), das eine entsprechend der Krümmung der Konkavität des Kreuzbeins gekrümmte Form aufweist, mit einer größeren Steifigkeit als die Außenhülle des Introduktors (4), mit einem Außendurchmesser und einer Länge, die derart sind, daß das Schutzrohr geeignet ist, über die Analöffnung eingeführt zu werden und sich zwischen der Analöffnung und bis vor die Anastomose zu erstrecken, und einem Innendurchmesser und einer Krümmung, entsprechend der Krümmung der Konkavität des Kreuzbeins, die derart sind, daß das Schutzrohr geeignet ist, den Introduktor zu enthalten und dessen Befördern zwischen der Analöffnung und der Anastomose zu ermöglichen, vorzugsweise ein Schutzrohr mit einem Außendurchmesser von 20 bis 40 mm und einer Länge von 10 bis 25 cm.

## Claims

1. A surgical device (1) for temporary protection of an anastomosis (5) in the colon (12, 13, 14), the rectum (11), or the anal passage (10), the device including:
a) a semi-rigid hollow longitudinal element called temporary anchor element (2), a first wall of which defines a surface of revolution about a longitudinal axis, including a substantially cylindrical main portion (2a) with a substantially circular section, and said first wall having an outside diameter that may be varied in a controlled manner between:
- a reduced outside diameter of at most 10 mm in a radially retracted configuration of said first wall; and
- a maximum outside diameter (D1) in a maximum radial expansion configuration of the main portion of said first wall in the range 20 mm to 40 mm, preferably in the range 25 mm to 33 mm; and
b) a sheath (3) having a flexible tubular wall fixed against, preferably around, said first wall of said anchor element (2);
• which device is **characterized in that**:
1) said first wall of said temporary anchor element (2) includes a said main portion of a greater diameter (2a) having a length (L1) of at least 50 mm, preferably in the range 70 mm to 150 mm; and
2) said sheath (3) has when at rest a length (L3) downstream of said anchor element (2) of at least 50 cm, preferably at least 1 m, and an outside diameter in the range 20 mm to 40 mm, preferably in the range 25 mm to 33 mm, and is made from a biocompatible elastomer material having a wall thickness in the range 0.05 mm to 1 mm.

2. A device according to claim 1, **characterized in that** said main portion (2a) of said first wall extends from the upstream longitudinal end (2₁) of said first wall as far as an appropriately shaped downstream end portion (2b) having a smaller diameter than the cylindrical main portion, the outside diameter (D2) of the downstream end (2₂) of said first wall in the maximum radial expansion configuration being in the range 20 mm to 35 mm and the length (L2) of said appropriately shaped end portion (2b) of the first wall being in the range 10 mm to 30 mm, preferably in the range 15 mm to 25 mm, the diameter of said end portion (2b) preferably decreasing progressively between said main portion and said downstream end of said first wall.

3. A device according to claim 2, **characterized in that** the diameter of said appropriately shaped downstream end portion (2b) is reduced by surrounding it with an elastomer material ring (3₁) having a diameter less than said diameter (D₁) of said main portion in said maximum radial expansion configuration.

4. A device according to any one of claims 1 to 3, **characterized in that**:
• said anchor element (2) is made from a material conferring on it radial elasticity properties such that it may be compressed radially in said retracted configuration and adopt said maximum radial expansion configuration (with diameter D₁) after the radial compression is released; and
• said sheath (3) is made from an elastomer-based biocompatible polymer material of the silicone or polyurethane type having a wall thickness in the range 0.1 mm to 0.5 mm and having radial and longitudinal elasticity properties, shape memory properties, and non-stick properties.

5. A device according to claim 4, **characterized in that** said temporary anchor element (2) is a stent-type enteral prosthesis said first wall of which is formed by a spiral elastomer or metal wire mesh.

6. A device according to any one of claims 1 to 5, **characterized in that** the connection between said sheath and said anchor element is produced by virtue of said sheath covering at least the external surface of the downstream end of said anchor element.

7. A device according to any one of claims 1 to 6, **characterized in that** said sheath is made from an elastomer-based biocompatible polymer material having the following properties:
• a Shore A hardness in the range 5 to 95, preferably in the range 50 to 70;
• a degree of remanent deformation on compression in the range 10% to 35%, preferably in the range 17% to 23%; and
• a coefficient of friction in the range 0.30 to 0.90, preferably in the range 0.35 to 0.45.

8. A device according to claim 6 or claim 7, **characterized in that** said anchor element (2) is a stent-type enteral prosthesis covered on at least its outside surface with a coating (24) of silicone or polyurethane type elastomer synthetic material, said elastomer coating being more flexible than the elastomer material of said sheath, and the connection between said sheath and said anchor element being produced by virtue of by said sheath covering said anchor element over only part (2b, 2c) of the length of said anchor element including at least its appropriately shaped downstream end (2b) of progressively reducing diameter.

9. A device according to any one of claims 1 to 8, **characterized in that** it further includes an introducer sheath (4) including:
• a tubular external envelope (4₁) that is adapted to contain said anchor element when compressed in said retracted configuration and to retain it inside the distal end of said external envelope and is sufficiently long also to contain said sheath (3), said external envelope preferably having a length of at least 110 cm, preferably at least 150 cm; and
• means (4₂, 4₃) for routing the distal end of said introducer sheath from the anal orifice (10) to said anchor site in the intestine upstream of the anastomosis (5); and
• preferably, means (4₄, 4₅) for disengaging said anchor element from the external envelope (4₁), preferably in the form of a buffer tube (4₄) including an abutment (4₅) at its distal end, where appropriate in contact with the longitudinal end of said anchor element, said sheath downstream of the anchor element surrounding said buffer tube (4₄) inside said external envelope.

10. A device according to any one of claims 1 to 9, **characterized in that** it further includes a protector tube (8), preferably a protector tube having an outside diameter in the range 20 mm to 40 mm and a length in the range 10 mm to 25 cm, that includes a portion (8a) of curved shape conforming to the curvature of the sacral concavity, is stiffer than the external envelope of the introducer sheath (4), has an outside diameter and length such that said protector tube may be introduced via the anal orifice and extend between the anal orifice and a point upstream of said anastomosis, and has an inside diameter and a curvature conforming to the curvature of the sacral concavity such that said protector tube is able to contain said introducer sheath and route it between the anal orifice and said anastomosis.
